(19) Europäisches Patentamt
European Patent Office
Office européen des brevets

(11) Veröffentlichungsnummer: **0 149 818**
**A2**

# (12) EUROPÄISCHE PATENTANMELDUNG

(21) Anmeldenummer: **84115778.7**

(22) Anmeldetag: **19.12.84**

(51) Int. Cl.⁴: **C 07 C 97/22**
C 07 D 295/10, C 07 D 213/74
C 07 D 307/14
//A61K31/135

(30) Priorität: **31.12.83 DE 3347657**

(43) Veröffentlichungstag der Anmeldung:
**31.07.85 Patentblatt 85/31**

(84) Benannte Vertragsstaaten:
**AT BE CH DE FR GB IT LI NL SE**

(71) Anmelder: **Troponwerke GmbH & Co. KG**
**Berliner Strasse 156**
**D-5000 Köln 80(DE)**

(72) Erfinder: **Fruchtmann, Romanis**
**Konrad-Adenaueer-Ufer 79/81**
**D 5000 Köln 1(DE)**

(72) Erfinder: **Dr. Horstmann, Harald**
**Claudiusweg 19**
**D 5600 Wuppertal 1(DE)**

(72) Erfinder: **Dr. Junge, Bodo**
**Spiekern 23**
**D 5600 Wuppertal 23(DE)**

(72) Erfinder: **Dr. Krupka, Udo**
**Oberer Eichweg 29**
**D 3550 Marburg(DE)**

(72) Erfinder: **Dr. Opitz, Wolfgang**
**Holzbachtalstrasse 60**
**D 5063 Overath(DE)**

(72) Erfinder: **Dr. Pelster, Bernhard**
**Pleisufer 6a**
**D-5205 St. Augustin 1(DE)**

(72) Erfinder: **Dr. Gauss, Walter**
**Ludwig-Aschoff-Strasse 4**
**D 5000 Köln 80(DE)**

(74) Vertreter: **Adrian, Albert, Dr. et al,**
**c/o BAYER AG Konzernverwaltung RP Patentabteilung**
**D-5090 Leverkusen 1, Bayerwerk(DE)**

(54) **Neue 1,4-Naphthochinonderivate, Verfahren zu ihrer Herstellung und ihre Verwendung als Arzneimittel.**

(57) Die vorliegende Erfindung betrifft neue Naphthochinonderivate, Verfahren zu ihrer Herstellung sowie ihre Verwendung als Wirkstoff in entzündungshemmend und antiarthritisch wirkenden Arzneimitteln. Die neuen Naphthochinonderivate entsprechen der Formel

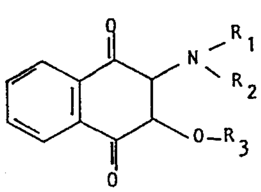

worin die Reste $R_1$, $R_2$ und $R_3$ die in der Beschreibung angegebene Bedeutung haben.

EP 0 149 818 A2

TROPONWERKE GmbH & Co. KG 5000 Köln 80
Je/Kü-c

# Neue 1,4-Naphthochinonderivate, Verfahren zu ihrer Herstellung und ihre Verwendung als Arzneimittel

Die vorliegende Erfindung betrifft neue Naphthochinonderivate, Verfahren zu ihrer Herstellung sowie ihre Verwendung als Wirkstoff in entzündungshemmend und antiarthritisch wirkenden Arzneimitteln. Die neuen Verbindungen entsprechen der allgemeinen Formel I

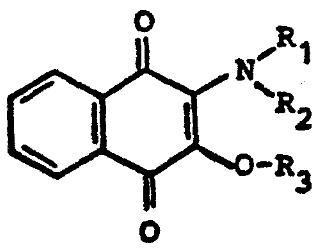

I

worin

$R_1$ für einen substituierten linearen oder verzweigten, gesättigten oder ungesättigten Alkylrest,

für einen gegebenenfalls substituierten gesättigten oder ungesättigten Cycloalkylrest,

für einen gegebenenfalls substituierten gesättigten oder ungesättigten Aralkylrest,

für einen substituierten Arylrest oder

für einen gegebenenfalls substituierten gesättigten oder ungesättigten heterocyclischen Rest steht,

$R_2$ dieselbe Bedeutung wie $R_1$ haben und gleich oder von diesem verschieden sein kann oder

für ein Wasserstoffatom,

für einen Acylrest, der im Falle, daß $R_1$ die Bedeutung von Benzyl hat, mehr als zwei C-Atome enthalten soll oder für einen Alkylsulfonyl- oder Arylsulfonylrest steht oder

$R_1$ und $R_2$ gemeinsam mit dem Stickstoffatom Bestandteil eines gegebenenfalls substituierten heterocyclischen Ringes sind und

$R_3$ für Wasserstoff sowie einen Acyl- oder Alkylsulfonyl- oder Arylsulfonylrest stehen kann.

Der substituierte lineare oder verzweigte, gesättigte oder ungesättigte Alkylrest $R_1$ steht bevorzugt für einen solchen mit 1 bis 6 C-Atomen, vorzugsweise 1 bis 4 C-Atomen, der bis zu zwei Doppelbindungen und der einen oder mehrere Substituenten aus der Reihe Amino-, Mono- oder Dialkylamino- mit bis zu vier C-Atomen je Alkylrest, Acylamino- bis zu vier C-Atomen, Alkoxy- mit bis zu vier

C-Atomen, Acyloxy- mit drei bis sechs C-Atomen, Alkylmercapto- mit bis zu vier C-Atomen, Carboxyl-, Carbamoyl-, Alkoxycarbonyl-, Hydroxyl-, Cyano-, Cycloalkylgruppen mit bis zu sechs C-Atomen sowie aromatische oder teilweise oder ganz gesättigte und gegebenenfalls einfach substituierte heterocyclische Ringe aus der Reihe Pyridyl, Furyl, Thienyl, Imidazolyl, Pyrolyl, Morpholinyl oder Indolyl trägt.

Der gegebenenfalls substituierte gesättigte oder ungesättigte Cycloalkylrest $R_1$ steht bevorzugt für einen solchen mit bis zu 6 C-Atomen, vorzugsweise 1 bis 4 C-Atomen, der durch einen Niedrigalkylrest mit 1 bis 3 C-Atomen oder einen Phenylrest substituiert sein und ein oder zwei Doppelbindungen enthalten kann.

Der gegebenenfalls substituierte Aralkylrest $R_1$ steht bevorzugtfür einen solchen, bei dem der Arylrest, bevorzugt ein Phenylrest, der durch Hydroxyl-, Alkyl- oder Alkoxygruppen mit bis zu drei C-Atomen, Halogenatome, bevorzugt Fluor- oder Chloratome, Nitro-, Cyano-, Carboxyl-, Carbamoyl-, Alkoxycarbonyl-, Alkylmercapto-, Amino-, Mono- oder Dialkylamino-, Acylamino- oder Sulfamoylgruppen substituiert sein kann, über eine Kohlenstoffkette mit bis zu drei C-Atomen, die gesättigt sein oder eine Doppelbindung enthalten und gegebenenfalls durch niedrige ($C_1$-$C_6$, vorzugsweise $C_1$-$C_4$) Alkyl-, Alkoxy-, Acyloxy- oder Hydroxylgruppen oder einen Phenylrest substituiert sein kann, an das Stickstoffatom gebunden ist.

Der substituierte Arylrest $R_1$ steht bevorzugt für einen Phenylrest, der durch niedrige Alkyl-, Alkylmercapto-, Acyl-, Acyloxy-, Acylamino-, Mono- oder Dialkylaminogruppen mit jeweils bis zu drei C-Atomen, Fluoratome, Hydroxyl-, Amino-, Cyano-, Carbamoyl- oder Alkoxycarbonylgruppen substituiert sein kann.

Der gegebenenfalls substituierte gesättigte oder ungesättigte heterocyclische Rest $R_1$ steht bevorzugt für einen aromatischen oder teilweise oder ganz gesättigten und gegebenenfalls einfach substituierten heterocyclischen fünf- oder sechsgliedrigen Ring, der als Heteroatome ein bis vier Stickstoffatome und maximal je ein Sauerstoff- oder Schwefelatom enthalten kann. Bevorzugt sind Pyridyl-, Thiazolyl-, Triazolyl-, Pyrazolyl-, Thiadiazolyl-, Pyrazinyl-, Isoxazolyl-, Isothiazolyl, Pyrimidyl- und Tetrazolylreste sowie gegebenenfalls deren ganz oder partiell hydrierte Analoga.

Der Acylrest $R_2$ steht bevorzugt für einen gerad- oder verzweigtkettigen, auch ungesättigten Acylrest mit bis zu sechs C-Atomen.

Der Alkylsulfonyl- oder Arylsulfonylrest $R_2$ steht bevorzugt für einen Methan-, Ethan-, Benzol- oder Toluolsulfonylrest.

Für den Fall, daß $R_1$ und $R_2$ gemeinsam mit dem Stickstoffatom Bestandteil eines heterocyclischen Ringes sind, handelt es sich bei den jeweiligen Heterocyclen

um gesättigte oder ungesättigte fünf- bis siebengliedrige Ringe, die außer dem als Verknüpfungsstelle dienenden Stickstoffatom noch ein weiteres Stickstoffatom und/oder maximal je ein Schwefel- oder Sauerstoffatom enthalten können. In diesem Sinne bevorzugte Heterocyclen sind z.B. Pyrrolidin, Pyrrolin, Piperin, Piperidin, Morpholin, Thiomorpholin, Piperazin sowie seine N-monosubstituierten Derivate. Bevorzugte Piperazinderivate sind Niedrigalkylderivate, wie z.B. der N-Methylpiperazinrest, Arylderivate, wie z.B. der N-2-Methoxyphenylpiperazinrest oder heterocyclische Derivate, wie z.B. der N-2-Pyridylpiperazinrest.

Der Acylrest $R_3$ steht bevorzugt für einen gerad- oder verzweigtkettigen, auch ungesättigten Acylrest mit ein bis sechs C-Atomen, der identisch mit dem Acylrest $R_2$ oder von ihm verschieden sein kann.

Der Alkylsulfonyl- oder Arylsulfonylrest $R_3$ steht bevorzugt für Methan-, Ethan-, Benzol- oder Toluolsulfonylrest und kann identisch mit dem Alkylsulfonyl- oder Arylsulfonylrest $R_2$ oder von ihm verschieden sein.

Erfindungsgemäß ist jede Kombination der angegebenen Reste, auch eine der bevorzugten mit den nicht bevorzugten Resten, möglich.

Die erfindungsgemäßen Verbindungen der allgemeinen Formel I, in denen

$R_1$ für einen substituierten linearen oder verzweigten, gesättigten oder ungesättigten Alkylrest,

für einen gegebenenfalls substituierten gesättigten oder ungesättigten Cycloalkylrest,

für einen gegebenenfalls substituierten gesättigten oder ungesättigten Aralkylrest,

für einen substituierten Arylrest oder

für einen gegebenenfalls substituierten gesättigten oder ungesättigten heterocyclischen Rest steht,

$R_2$ dieselbe Bedeutung wie $R_1$ hat und gleich oder von diesem verschieden ist oder für ein Wasserstoffatom steht oder

$R_1$ und $R_2$ gemeinsam mit dem Stickstoffatom Bestandteil eines gegebenenfalls substituierten heterocyclischen Ringes sind und

$R_3$ für Wasserstoff steht

werden erhalten, indem man

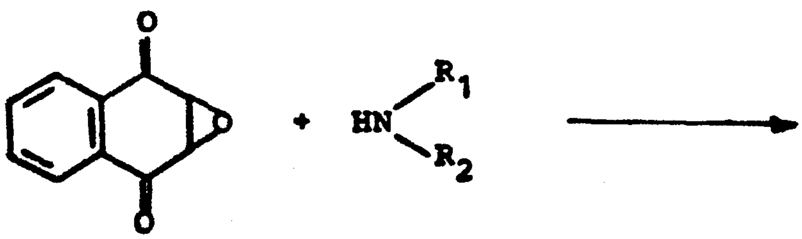

II III

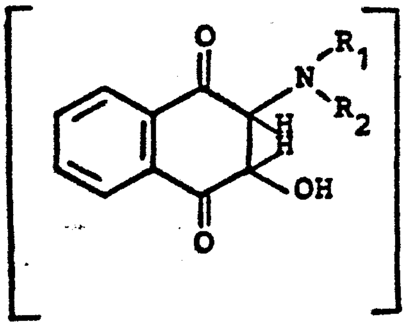

IV

IV 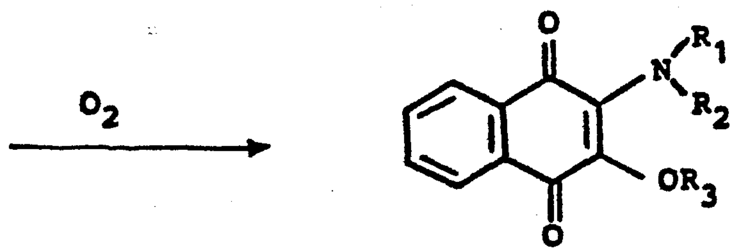


2-Epoxi-1,4-dioxotetralin II mit Aminen der allgemeinen Formel III, in der

$R_1$ für einen substituierten linearen oder verzweigten, gesättigten oder ungesättigten Alkylrest,

für einen gegebenenfalls substituierten gesättigten oder ungesättigten Cycloalkylrest,

für einen gegebenenfalls substituierten gesättigten oder ungesättigten Aralkylrest,

für einen substituierten Arylrest oder

für einen gegebenenfalls substituierten gesättigten oder ungesättigten heterocyclischen Rest steht,

$R_2$ dieselbe Bedeutung wie $R_1$ hat und gleich oder von diesem verschieden ist oder für ein Wasserstoffatom steht oder

$R_1$ und $R_2$ gemeinsam mit dem Stickstoffatom Bestandteil eines gegebenenfalls substituierten heterocyclischen Ringes sind,

in einem geeigneten Lösungsmittel umsetzt. Dabei werden zunächst die Verbindungen der allgemeinen Formel IV gebildet, die nicht isoliert, sondern durch Sauerstoff, vorzugsweise Luftsauerstoff, direkt zu den erfindungsgemäßen Verbindungen der allgemeinen Formel I, in denen

$R_1$ für einen substituierten linearen oder verzweigten, gesättigten oder ungesättigten Alkylrest,

für einen gegebenenfalls substituierten gesättigten oder ungesättigten Cycloalkylrest,

für einen gegebenenfalls substituierten gesättigten oder ungesättigten Aralkylrest,

für einen substituierten Arylrest oder

für einen gegebenenfalls substituierten gesättigten oder ungesättigten heterocyclischen Rest steht,

$R_2$ dieselbe Bedeutung wie $R_1$ und gleich oder von diesem verschieden ist oder für ein Wasserstoffatom steht oder

$R_1$ und $R_2$ gemeinsam mit dem Stickstoffatom Bestandteil eines gegebenenfalls substituierten heterocyclischen Ringes sind und

$R_3$ für Wasserstoff steht,

oxidiert werden.

Für die Durchführung der Reaktion sind polar protische Lösungsmittel, wie niedrige Alkohole, z.B. Ethanol, polar aprotische Lösungsmittel, wie cyclische Ether, besonders Dioxan sowie überschüssiges Amin III selbst geeignet. Die Reaktion wird bei Temperaturen zwischen 0°C und der Rückflußtemperatur des jeweils verwendeten Lösungsmittels durchgeführt, bevorzugte Reaktionstemperaturen liegen zwischen 20°C und der jeweiligen Rückflußtemperatur.

In Fällen, in denen das Amin III außer der Aminfunktion eine weitere funktionelle Gruppe enthält, kann es nötig sein, diese vor der Umsetzung mit dem Epoxid II nach literatu bekannten Methoden zu schützen und die Schutzgruppe nach der Reaktion mit dem Epoxid wieder abzuspalten.

Zu den erfindungsgemäßen Verbindungen der allgemeinen Formel I, in denen

$R_1$ für einen substituierten linearen oder verzweigten, gesättigten oder ungesättigten Alkylrest,

für einen gegebenenfalls substituierten gesättigten oder ungesättigten Cycloalkylrest,

für einen gegebenenfalls substituierten gesättigten oder ungesättigten Aralkylrest,

für einen substituierten Arylrest oder

für einen gegebenenfalls substituierten gesättigten oder ungesättigten heterocyclischen Rest stehen,

$R_2$ und $R_3$ für einen Acyl-, Alkylsulfonyl- oder Arylsulfonylrest stehen,

gelangt man, indem man Verbindungen der allgemeinen Formel I, in denen

$R_1$ für einen substituierten linearen oder verzweigten, gesättigten oder ungesättigten Alkylrest,

für einen gegebenenfalls substituierten gesättigten oder ungesättigten Cycloalkylrest,

für einen gegebenenfalls substituierten gesättigten oder ungesättigten Aralkylrest,

für einen substituierten Arylrest oder

für einen gegebenenfalls substituierten gesättigten oder ungesättigten heterocyclischen Rest steht,

$R_2$ und $R_3$ für Wasserstoff stehen,

mit Säurederivaten, geeignet sind Halogenide und Anhydride, die diese Acyl-, Alkylsulfonyl- oder Arylsulfonylreste enthalten, nach literaturbekannten Methoden umsetzt.

Durch gezielte unvollständige Umsetzung werden bei dieser Reaktion auch Verbindungen der allgemeinen Formel I erhalten, in denen

$R_1$ für einen substituierten linearen oder verzweigten, gesättigten oder ungesättigten Alkylrest,

für einen gegebenenfalls substituierten gesättigten oder ungesättigten Cycloalkylrest,

für einen gegebenenfalls substituierten gesättigten oder ungesättigten Aralkylrest,

für einen substituierten Arylrest oder

für einen gegebenenfalls substituierten gesättigten oder ungesättigten heterocyclischen Rest steht,

$R_2$ für einen Acyl-, Alkylsulfonyl- oder Arylsulfonylrest und

$R_3$ für Wasserstoff steht.

Diese Verbindungen werden ebenfalls erhalten, wenn man Verbindungen der allgemeinen Formel I, in denen

$R_1$ für einen substituierten linearen oder verzweigten, gesättigten oder ungesättigten Alkylrest,

für einen gegebenenfalls substituierten gesättigten oder ungesättigten Cycloalkylrest,

für einen gegebenenfalls substituierten gesättigten oder ungesättigten Aralkylrest,

für einen substituierten Arylrest oder

für einen gegebenenfalls substituierten gesättigten oder ungesättigten heterocyclischen Rest steht,

$R_2$ und $R_3$ für einen Acyl-, Alkylsulfonyl- oder Arylsulfonylrest stehen,

nach literaturbekannten Methoden partiell verseift.

Nach den vorbeschriebenen Methoden wurden die im folgenden aufgeführten besonders bevorzugten erfindungsgemäßen Verbindungen der allgemeinen Formel I hergestellt:

Verb. 1 : 2-Benzylamino-3-hydroxy-naphthochinon-1,4
Verb. 2 : 2-(4-Chlorbenzylamino)-3-hydroxy-naphthochinon-1,4
Verb. 3 : 2-(3-Chlorbenzylamino)-3-hydroxy-naphthochinon-1,4
Verb. 4 : 2-(2-Chlorbenzylamino)-3-hydroxy-naphthochinon-1,4
Verb. 5 : 2-(4-Morpholinyl)-3-hydroxy-naphthochinon-1,4
Verb. 6 : 2-(2-Methoxybenzylamino)-3-hydroxy-naphthochinon-1,4
Verb. 7 : 2-(1-Piperidinyl)-3-hydroxy-naphthochinon-1,4
Verb. 8 : 2-(2-Phenylethylamino)-3-hydroxy-naphthochinon-1,4
Verb. 9 : 2-(1-Pyrrolidinyl)-3-hydroxy-naphthochinon-1,4
Verb. 10: 2-(4-Thiomorpholinyl)-3-hydroxy-naphthochinon-1,4
Verb. 11: 2-(2-Pyridylamino)-3-hydroxy-naphthochinon-1,4
Verb. 12: 2-(4-Methylpiperazin-1-yl)-3-hydroxy-naphthochinon-1,4
Verb. 13: 2-((N-Acetyl-N-benzyl)amino)-3-acetoxy-naphthochinon-1,4
Verb. 14: 2-(4-Benzyloxy-phenylamino)-3-hydroxy-naphthochinon-1,4

Verb. 15: 2-(4-Hydroxy-phenylamino)-3-hydroxy-naphthochinon-1,4

Verb. 16: 2-(4-(2-Methoxyphenyl)-piperazin-1-yl)-3-hydroxy-naphthochinon-1,4

Verb. 17: 2-(2-Furylmethylamino)-3-hydroxy-naphthochinon-1,4

Verb. 18: 2-(N-Acetyl-N-(2-furylmethyl)-amino)-3-acetoxy-naphthochinon-1,4

Verb. 19: 2-(N-Acetyl-N-(2-furylmethyl)amino)-3-hydroxy-naphthochinon-1,4

Es wurde gefunden, daß die erfindungsgemäßen Verbindungen der allgemeinen Formel I ausgezeichnete entzündungshemmende und antiarthritische Aktivität bei geringer Allgemeintoxizität aufweisen.

Die Verbindungen der allgemeinen Formel I hemmen das durch Carrageenan ausgelöste Ödem der Rattenpfote stark. Zum Nachweis diente die Methode von Winter et al., Proc. Soc. Exp. Bil. Med. 111, 544 (1962), mit der Abwandlung, daß zur Ödemauslösung reines $\lambda$-Carrageenan als 0,25 % Suspension in physiologischer Kochsalzlösung verwendet wurde. Beispielhaft wurde in diesem Testmodell für Verb. 1 die $DE_{50}$ = 0,5 mg/kg p.o ermittelt.

Wie die meisten NSAID (non steroidal antiinflammatory drugs) hemmen auch die meisten Verbindungen der allgemeinen Formel I die Metabolisierung der Arachidonsäure über den Cyclooxygenaseweg mehr oder weniger stark

(Methode von Brune et al., Naunyn-Schmiedeberg's Arch. Pharmacol. 315, 269 (1981)). Die $IC_{50}$ für Verb. 1 in diesem Test beträgt 2.6 x $10^{-6}$ Mol/l.

Überraschenderweise hemmen darüber hinaus alle Verbindungen der allgemeinen Formel I auch die Metabolisierung der Arachidonsäure über den Lipoxygenaseweg außerordentlich stark.

Die Meßmethode beruht auf der Bestimmung der Freisetzung von $LTB_4$ aus PMN-Leukozyten der Ratte nach Stimulation mit Ca-Ionophor mittels reversed phase-HPLC (Borgeat et al., Proc. Natl. Acad. Sci. USA 76, 2148 (1979), Ford-Hutchinson et al., Brit. J. Pharmacol. 76, 215 (1982)). Als Beispiel wurde die $IC_{50}$ von Verb. 1 in diesem Test mit 4.4 x $10^{-6}$ Mol/l bestimmt.

Darüber hinaus konnte für eine Reihe von Verbindungen der allgemeinen Formel I nachgewiesen werden, daß sie die durch das chemotaktisch wirkende FMLP stimulierte Aggregation von PMN-Leukozyten hemmen und dadurch Anteil an der entzündungshemmenden Wirkung haben (Methode von Cunningham et al., Agents & Actions 11, 583 (1981)). Für Verb. 1 ist die $IC_{50}$ in diesem Test 3.7 x $10^{-5}$ Mol/l.

TP 72

Zur vorliegenden Erfindung gehören pharmazeutische Zubereitungen, die neben nichttoxischen, inerten, pharmazeutisch geeigneten Tägerstoffen, einen oder mehrere erfindungsgemäße Wirkstoffe enthalten, oder die aus einem oder mehreren erfindungsgemäßen Wirkstoffen bestehen, sowie Verfahren zur Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entspricht. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagsdosis entspricht.

Unter nichttoxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirktoffe neben den üblichen Trägerstoffen enthalten, wie Füll- und Streckmittel (z.B. Stärken, Milchzucker, Rohrzucker, Glukose, Mannit und Kieselsäure), Bindemittel (z.B. Carboxymethylcellulose, Alignate, Gelatine, Polyvinylpyrrolidon), Fuechthaltemittel (z.B. Glycerin), Sprengmittel (z.B. Agar-Agar , Calciumcarbonat und Natriumbicarbonat), Lösungsverzögerer (z.B. Paraffin) und Resorptionsbeschleuniger (z.B. quaternäre Ammoniumverbindungen), Adsorptionsmittel (z.B. Kaolin und Bentonit) und Gleitmittel (z.B. Talkum, Calcium- und Magnesiumstearat und feste Polyethylenglykole) oder Gemische der aufgeführten Stoffe.

Die Tabletten, Dragees, Kapseln, Pillen und Granulate können mit den üblichen, gegebenenfalls Opakisierungsmittel enthaltenden Überzügen und Hüllen versehen sein und auch so zusammgensetzt sein, daß sie den oder die Wirkstoffe nur oder bevorzugt in einem bestimmten Teil des Intestinaltraktes, gegebenenfalls verzögert, abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen zur Erzielung eines Retardeffektes.

Suppositorien können neben dem oder den Wirkstoffen die üblichen wasserlöslichen oder wasserunlöslichen Trägerstoffe enthalten, z.B. Polyethylenglykole, Fette z.B. Kakaofett und höhere Ester (z.B. $C_{14}$-Alkohol mit $C_{16}$-Fettsäure) oder Gemische dieser Stoffe.

Salben, Pasten, Cremes und Gele können nebem dem oder den Wirkstoffen die üblichen Trägerstoffe enthalten (z.B. tierische und pflanzliche Fette, Wachse, Paraffine, Stärke, Tragant) oder Gemische dieser Stoffe.

Puder und Sprays können neben dem oder den Wirkstoffen die üblichen Trägerstoffe enthalten (z.B. Milchzucker, Talkum, Kieselsäure, Aluminiumhydroxid) oder Gemische dieser Stoffe. Sprays können zusätzlich die üblichen Treibmittel enthalten.

Lösungen und Emulsionen können neben dem oder den Wirkstoffen die üblichen Trägerstoffe wie Lösungsmittel, Lösungsvermittler und Emulgatoren, z.B. Wasser, Ethylalkohol, Isopropylalkohol, Ethylcarbonat, Ethylacetat, Benzylalkohol, Benzylbenzoat, Propylenglykol, Öle, insbesondere Baumwollsaatöl, Erdnußöl, Maiskeimöl, Olivenöl und Sesamöl, Glycerin, Polyethylenglykole oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel (z.B. Wasser, Ethylalkohol, Propylenglykol), Suspendiermittel (z.B. ethoxylierte Isostearylalkohole, Polyoxyethylensorbit- und Sorbitanester, mikrokristalline Cellulose) oder Gemische dieser Stoffe enthalten.

Die genannten Formulierungsformen können auch Färbemittel, Konservierungsstoffe sowie geruchs- und geschmacksverbessernde Zusätze, z.B. Pfefferminzöl und Eukalyptusöl und Süßmittel, z.B. Saccharin, enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-% der Gesamtmischung, vorhanden sein.

Die oben aufgeführten pharmazeutischen Zubereitungen können außer den erfindungsgemäßen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Zur vorliegenden Erfindung gehört auch die Verwendung der erfindungsgemäßen Wirkstoffe sowie von pharmazeutischen Zubereitungen, die einen oder mehrere erfindungsgemäße Wirkstoffe enthalten, in der Human- und Veterinärmedizin zur Verhütung, Besserung und/oder Heilung entzündlicher Prozesse im menschlichen und tierischen Organismus.

Die Wirkstoffe oder die pharmazeutischen Zubereitungen können cutan, oral, parenteral, intraperitoneal und/oder rectal, vorzugsweise oral oder cutan, appliziert werden.

Im allgemeinen ist es in der Humanmedizin als vorteilhaft anzusehen, den oder die erfindungsgemäßen Wirkstoffe in Gesamtmengen von etwa 0,1 bis 200, vorzugsweise 0,1 bis 70 mg je 24 Stunden, gegebenenfalls in Form mehrerer Einzelgaben zur Erzielung der gewünschten Ergebnisse, zu verabreichen.

Es kann jedoch erforderlich sein, von den genannten Dosierungen abzuweichen, und zwar in Abhängigkeit von der Art und dem Körpergewicht des zu behandelnden Objektes, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der obengenannten Menge Wirkstoff auszukommen, während in anderen Fällen die oben angeführte Wirkstoffmenge überschritten werden muß.

Die Festlegung der jeweils erforderlichen optimalen Dosierung und Applikationsart der Wirkstoffe kann durch jeden Fachmann aufgrund seines Fachwissens leicht erfolgen.

Die vorliegende Erfindung soll durch die nachfolgenden Beispiele noch weiter erläutert werden.

## Beispiel 1

2-Benzylamino-3-hydroxy-naphthochinon-1,4 (Verb. 1)

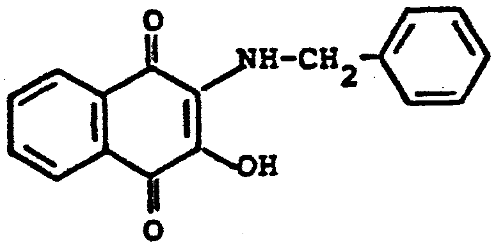

Eine Suspension von 17,5 g (0,1 Mol) 2-Epoxi-1,4-dioxotetralin in 85 ml Dioxan wird unter Rühren und Luftzutritt mit 32,1 g (0,3 Mol) Benzylamin versetzt, wobei die Temperatur 45°C nicht übersteigen sollte. Man rührt noch 90 Minuten bei Raumtemperatur, säuert das Reaktionsgemisch durch Zugabe von 450 ml n Salzsäure an, filtriert den ausgefallenen violettschwarzen Niederschlag ab und kristallisiert ihn aus Aceton um.

Ausbeute: 5,8 g (20,7 % der Theorie)
Schmelzpunkt: 140 - 143°C.

In Analogie dazu wurden erhalten:

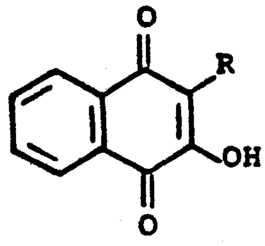

| Verbind. | R | Reaktions-temperat. | Reaktions-zeit | Aufarbeitung | Ausbeute | Schmp. |
|---|---|---|---|---|---|---|
| 2 | 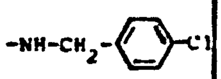 | Raumtemp. | 90 Min. | ansäuern, Niederschlag aus Aceton umkristallisieren | 14.3% | 203-5°C |
| 3 | 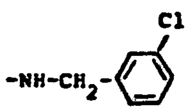 | Raumtemp. | 90 Min. | ansäuern, Niederschlag aus Aceton umkristallisieren | 17.3% | 175-6°C |
| 4 | 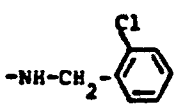 | Raumtemp. | 90 Min. | ansäuern, Niederschlag aus Aceton umkristallisieren | 25.5% | 172-4°C |
| 5 | 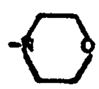 | Raumtemp. | 90 Min. | abdestillieren, Rückstand mit Essigester an Kieselgel chromatographieren, aus Dichlormethan/Petrolether kristallisieren | 45.4% | 172-4°C |
| 6 | 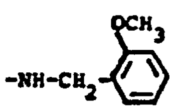 | Raumtemp. | 90 Min. | ansäuern, mit Dichlormethan extrahieren, Extrakt nach Eindampfen mit Dichlormethan an Kieselgel chromatographieren | 6.7% | 135-6°C |
| 7 | 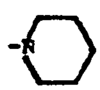 | Raumtemp. | 90 Min. | abdestillieren, Rückstand mit Essigester an Kieselgel chromatographieren, aus Dichlormethan/Petrolether kristallisieren | 36% | 187-8°C |
| 8 | 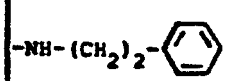 | Raumtemp. | 90 Min. | ansäuern, Niederschlag aus Aceton umkristallisieren | 18.1% | 170-1°C |

TP 72

| Verbind. | R | Reaktions-temperat. | Reaktions-zeit | Aufarbeitung | Ausbeute | Schmp. |
|---|---|---|---|---|---|---|
| 9 | 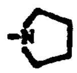 | 50°C | 15 Min. | abdestillieren, Rückstand mit Essigester über Kieselgel filtrieren, Eindampfrückstand mit Petrolether ausrühren | 13.4% | 180-2°C |
| 10 | 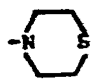 | Raumtemp. | 65 Std. | abdestillieren, Rückstand mit Essigester über Kieselgel filtrieren, Eindampfrückstand mit Petrolether ausrühren | 15% | 196-8°C |
| 11 | 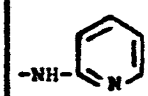 | Raumtemp.<br>50 - 60°C | 120 Min.<br>30 Min. | mit Wasser verdünnen, mit Dichlormethan extrahieren, Eindampfrückstand aus Aceton kristallisieren | 21.6% | 304-6°C |
| 12 | 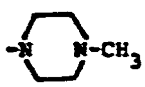 | Raumtemp. | 65 Std. | abdestillieren, Rückstand mit Ethanol/Wasser 7:3 an Kieselgel chromatographieren, Eindampfrückstand mit Ethanol/Wasser ausrühren | 43.7% | 209-10°C |
| 14 | 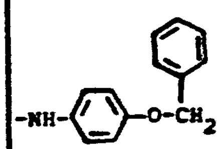 | Rückfluß | 3 Std. | mit Diethylether/Petrolether 2:1 fällen | 59.6% | 200-1°C |
| 16 | 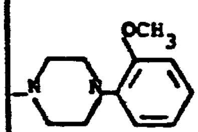 | Raumtemp. | 3 Std. | mit Diethylether fällen, Niederschlag mit Essigester an Kieselgel chromatografieren, Eindampfrückstand mit Petrolether ausrühren | 37.8% | 166-8°C |
| 17 | 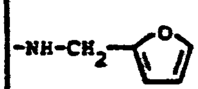 | Raumtemp. unter $N_2$<br>unter Luftzutritt | 120 Min.<br>15 Min. | ansäuern, Niederschlag mit Ethylenchlorid an Kieselgel chromatographieren | 20% | 141-4°C |

0149818

Beispiel 2

2-(N-Acetyl-N-(2-furylmethyl)amino)-3-acetoxy-naphthochionon-1,4 (Verb. 18)

Eine Mischung aus 2,7 g (10 mMol) 2-(2-Furylmethylamino)-3-hydroxy-naphthochinon-1,4, 4 ml Pyridin und 5,6 ml Essigsäureanhydrid wird 30 Minuten auf 90°C erwärmt. Der nach Abdampfen des Lösungsmittel verbleibende Rückstand wird mit Cyclohexan/Essigester 1:1 an Kieselgel chromatographiert. Die produktenthaltenden Fraktionen werden eingedampft und der Rückstand mit Ether ausgerührt.

Ausbeute: 770 mg (21,8 % der Theorie)
Schmelzpunkt: 94 - 6°C.

Analog wird aus 2-Benzylamino-3-hydroxy-naphthochinon-1,4 erhalten:

2- (-Acetyl-N-benzyl amino)-3-acetoxy-naphthochinon-1,4 (Verb. 13)

Ausbeute: 6,1 % der Theorie; orangefarbenes Harz.

Beispiel 3

2-(N-Acetyl-N(2-furylmethyl)amino)-3-hydroxy-naphthochinon-1,4 (Verb. 19)

Eine Mischung aus 2,7 g (10 mMol) 2-(2-Furylmethylamino)-3-hydroxy-naphthochinon-1,4, 4 ml Pyridin und 5,6 ml Essigsäureanhydrid wird 30 Minuten auf 90°C erwärmt und das Lösungsmittel abgedampft. Der Rückstand wird in Dichlormethan aufgenommen und mit gesättigter $KHCO_3$-Lösung extrahiert. Die wäßrige Phase wird mit Salzsäure angesäuert und mit Dichlormethan extrahiert. Nach Verdampfen des Dichlormethans verbleibt das Produkt als Rückstand.

Ausbeute: 1 g (32,2 % der Theorie)
Schmelzpunkt: 153 - 4°C.

Beispiel 4

2-(4-Hydroxyphenylamino)-3-hydroxy-naphthochinon-1,4 (Verb. 15)

3,71 g (10 mMol) 2-(4-Benzyloxyphenylamino)-3-hydroxy-naphthochinon-1,4 werden mit 40 ml einer 37 %igen Lösung von HBr in Eisessig übergossen und 15 Minuten bei 40°C aufbewahrt. Man versetzt mit Wasser und reinigt den ausgefallenen Niederschlag durch Chromatographie mit Essigester/Dichlormethan 1:4 an Kieselgel.

Ausbeute: 1,15 g (41 % der Theorie)
Schmelzpunkt: 205 - 7°C.

TP 72

Europäische Patentanmeldung Nr. 84115778.7
Anlage zur Eingabe vom 19. April 1985 / Ansprüche für BE, DE, GB, FR, NL, IT, SE, CH

Patentansprüche

1. Verbindungen der allgemeinen Formel I

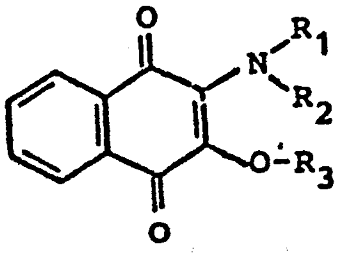

I

worin

$R_1$ für einen substituierten linearen oder verzweigten, gesättigten oder ungesättigten Alkylrest,

für einen gegebenenfalls substituierten gesättigten oder ungesättigten Cycloalkylrest,

für einen gegebenenfalls substituierten gesättigten oder ungesättigten Aralkylrest,

für einen substituierten Arylrest oder

für einen gegebenenfalls substituierten gesättigten oder ungesättigten heterocyclischen Rest steht,

$R_2$ dieselbe Bedeutung wie $R_1$ haben und gleich oder von diesem verschieden sein kann oder

für ein Wasserstoffatom,

für einen Acylrest, der im Falle, daß $R_1$ die Bedeutung von Benzyl hat, mehr als zwei C-Atome

enthalten soll oder für einen Alkylsulfonyl- oder Arylsulfonylrest steht oder

$R_1$ und $R_2$ gemeinsam mit dem Stickstoffatom Bestandteil eines gegebenenfalls substituierten heterocyclischen Ringes sind und

$R_3$ für Wasserstoff sowie einen Acyl- oder Alkylsulfonyl- oder Arylsulfonylrest stehen kann.

2. Verbindungen der allgemeinen Formel I

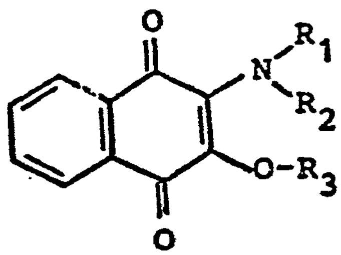

I

worin

$R_1$ für einen Alkylrest mit bis zu 6 C-Atomen, der durch Amino-, mono-, Dialkyl-, Acylamino, Alkoxy, Acyloxy, Alkylmercapto-, Carboxyl-, Carbamoyl-, Alkoxycarbonyl-, Hydroxyl-, Cyano-, Cycloalkyl, aromatische oder teilweise oder ganz gesättigte Heterocyclen aus der Reihe Pyridyl, Furyl, Thienyl, Imidazolyl, Pyrolyl, Morpholinyl oder Indolyl substituiert sein kann,

für einen Cycloalkylrest mit bis zu 6 C-Atomen, der durch einen Alkylrest mit 1 - 3 C-Atomen oder Phenylrest substituiert sein kann oder ein oder zwei Doppelbindungen enthalten kann,

für einen Aralkylrest steht, bei dem der Arylrest, vorzugsweise ein Phenylrest, durch Hydroxyl-, Alkyl- oder Alkoxygruppen mit bis zu 3 C-Atomen, Halogenatome, Nitro-, Cyano-, Car-boxyl-, Carbamoyl-, Alkoxycarbonyl-, Alkyl-mercapto-, Amino-, Mono- oder Dialkylamino, Acylamino oder Sulfamoylgruppen substituiert sein kann über eine Kohlenstoffkette mit bis zu 3 C-Atomen, die gesättigt sein oder eine Doppelbindung enthalten und gegebenenfalls durch niedrige Alkyl-, Alkoxy-, Acyloxy- oder Hydroxylgruppen oder einen Phenylrest substituiert sein kann, an das Stickstoffatom gebunden ist,

für einen Arylrest, bevorzugt einen Phenylrest, der durch niedrige Alkyl-, Alkylmercapto-, Acyl-, Acyloxy-, Acylamino-, Mono-oder Dialkylaminogruppen mit jeweils bis zu 3 C-Atomen, Fluoratome, Hydroxyl-, Amino-, Cyano-, Carbamoyl- oder Alkoxycarbonylgruppen substituiert sein kann,

für einen aromatischen oder teilweise oder ganz ungesättigten und gegebenenfalls einfach substituierten heterocyclischen fünf- oder sechsgliedrigen Ring, der als Heteroatome bis zu 4 Stickstoffatome und maximal je ein Sauerstoff- oder Schwefelatom enthalten kann und

$R_2$ dieselbe Bedeutung wie $R_1$ besitzen und gleich oder verschieden von diesem sein kann und für Wasserstoff oder einen geraden oder verzweigten, auch ungesättigten Acylrest mit bis zu 6 C-Atomen oder für einen Methan-, Ethan-, Benzol- oder Toluolsulfonylrest steht und

$R_1$ und $R_2$ gemeinsam mit dem Stickstoffatom Bestandteil eines gegebenenfalls substituierten heterocyclischen Ringes sind und

$R_3$ für Wasserstoff, einen geraden oder verzweigten, auch ungesättigten Acylrest mit 1 bis 6 C-Atomen, einen Methan-, Ethan-, Benzol- oder Toluolsulfonylrest steht.

3. 2-Benzylamino-3-hydroxy-naphthochinon-1,4.

4. 2-(N-Acetyl-N-(2-furylmethyl)amino)-3-acetoxy-naphthochinon-1,4.

5. Verbindungen der allgemeinen Formel I

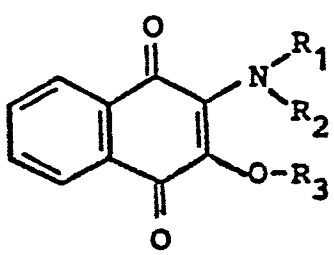

worin

$R_1$ für einen substituierten linearen oder verzweigten, gesättigten oder ungesättigten Alkylrest

für einen gegebenenfalls substituierten gesättigten oder ungesättigten Cycloalkylrest,

für einen gegebenenfalls substituierten gesättigten oder ungesättigten Aralkylrest,

für einen substituierten Arylrest oder

für einen gegebenenfalls substituierten gesättigten oder ungesättigten heterocyclischen Rest steht,

$R_2$ dieselbe Bedeutung wie $R_1$ haben und gleich oder von diesem verschieden sein kann oder

für ein Wasserstoffatom,

für einen Acylrest, der im Falle, daß $R_1$ die Bedeutung von Benzyl hat, mehr als zwei C-Atome enthalten soll oder für einen Alkylsulfonyl- oder Arylsulfonylrest steht oder

$R_1$ und $R_2$ gemeinsam mit dem Stickstoffatom Bestandteil eines gegebenenfalls substituierten heterocyclischen Ringes sind und

$R_3$ für Wasserstoff sowie einen Acyl- oder Alkylsulfonyl- oder Arylsulfonylrest stehen kann,

zur Verwendung bei der Bekämpfung von Krankheiten.

6. Verbindungen der allgemeinen Formel I

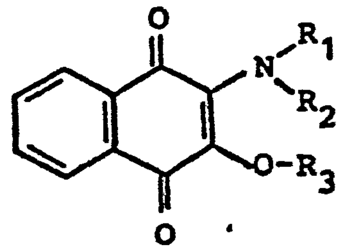

worin

$R_1$ für einen Alkylrest mit bis zu 6 C-Atomen, der durch Amino-, mono-, Dialkyl-, Acylamino, Alkoxy, Acyloxy, Alkylmercapto-, Carboxyl-, Carbamoyl-, Alkoxycarbonyl-, Hydroxyl-, Cyano-, Cycloalkyl, aromatische oder teilweise oder ganz gesättigte Heterocyclen aus der Reihe Pyridyl, Furyl, Thienyl, Imidazolyl, Pyrolyl, Morpholinyl oder Indolyl substituiert sein kann,

für einen Cycloalkylrest mit bis zu 6 C-Atomen, der durch einen Alkylrest mit 1 - 3 C-Atomen oder Phenylrest substituiert sein kann oder ein oder zwei Doppelbindungen enthalten kann, für einen Aralkylrest steht, bei dem der Arylrest, vorzugsweise ein Phenylrest, durch Hydroxyl-, Alkyl- oder Alkoxygruppen mit bis zu 3 C-Atomen, Halogenatome, Nitro-, Cyano-, Carboxyl-, Carbamoyl-, Alkoxycarbonyl-, Alkylmercapto-, Amino-, Mono- oder Dialkylamino, Acylamino oder Sulfamoylgruppen substituiert sein kann über eine Kohlenstoffkette mit bis zu 3 C-Atomen, die gesättigt sein oder eine Doppelbindung enthalten und gegebenenfalls

durch niedrige Alkyl-, Alkoxy-, Acyloxy- oder Hydroxylgruppen oder einen Phenylrest substituiert sein kann, an das Stickstoffatom gebunden ist,

für einen Arylrest, bevorzugt einen Phenylrest, der durch niedrige Alkyl-, Alkylmercapto-, Acyl-, Acyloxy-, Acylamino-, Mono- oder Dialkylaminogruppen mit jeweils bis zu 3 C-Atomen, Fluoratome, Hydroxyl-, Amino-, Cyano-, Carbamoyl- oder Alkoxycarbonylgruppen gesättigt sein kann,

für einen aromatischen oder teilweise oder ganz ungesättigten und gegebenenfalls einfach substituierten heterocyclischen fünf- oder sechsgliedrigen Ring, der als Heteroatome bis zu 4 Stickstoffatome und maximal je ein Sauerstoff- oder Schwefelatom enthalten kann und

$R_2$ dieselbe Bedeutung wie $R_1$ besitzen und gleich oder verschieden von diesem sein kann und für Wasserstoff oder einen geraden oder verzweigten, auch ungesättigten Acylrest mit bis zu 6 C-Atomen oder für einen Methan-, Ethan-, Benzol- oder Toluolsulfonylrest steht und

$R_1$ und $R_2$ gemeinsam mit dem Stickstoffatom Bestandteil eines gegebenenfalls substituierten heterocyclischen Ringes sind und

$R_3$ für Wasserstoff, einen geraden oder verzweigten, auch ungesättigten Acylrest mit 1 bis 6 C-Atomen, einen Methan-, Ethan-, Benzol- oder Toluolsulfonylrest steht,

zur Verwendung bei der Bekämpfung von Krankheiten.

7. Mittel, enthaltend eine oder mehrere Verbindungen der allgemeinen Formel I

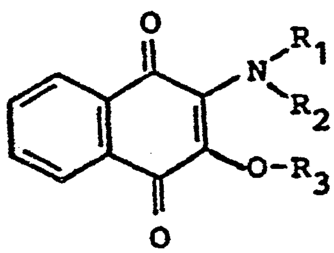

worin

$R_1$ für einen substituierten linearen oder verzweigten, gesättigten oder ungesättigten Alkylrest,

für einen gegebenenfalls substituierten gesättigten oder ungesättigten Cycloalkylrest,

für einen gegebenenfalls substituierten gesättigten oder ungesättigten Aralkylrest,

für einen substituierten Arylrest oder

für einen gegebenenfalls substituierten gesättigten oder ungesättigten heterocyclischen Rest steht,

$R_2$ dieselbe Bedeutung wie $R_1$ haben und gleich oder von diesem verschieden sein kann oder

für ein Wasserstoffatom,

für einen Acylrest, der im Falle, daß $R_1$ die Bedeutung von Benzyl hat, mehr als zwei C-Atome enthalten soll oder für einen Alkylsulfonyl- oder Arylsulfonylrest steht oder

$R_1$ und $R_2$ gemeinsam mit dem Stickstoffatom Bestandteil eines gegebenenfalls substituierten heterocyclischen Ringes sind und

$R_3$ für Wasserstoff sowie einen Acyl- oder Alkylsulfonyl- oder Arylsulfonylrest stehen kann.

8. Verwendung von Verbindungen der allgemeinen Formel I

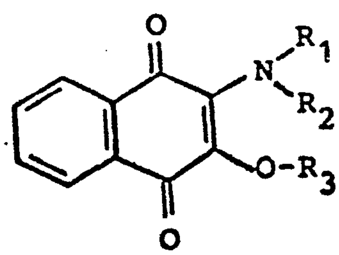

worin

$R_1$ für einen substituierten linearen oder verzweigten, gesättigten oder ungesättigten Alkylrest,

für einen gegebenenfalls substituierten gesättigten oder ungesättigten Cycloalkylrest,

für einen gegebenenfalls substituierten gesättigten oder ungesättigten Aralkylrest,

für einen substituierten Arylrest oder

für einen gegebenenfalls substituierten gesättigten oder ungesättigten heterocyclischen Rest steht,

$R_2$ dieselbe Bedeutung wie $R_1$ haben und gleich oder von diesem verschieden sein kann oder

für ein Wasserstoffatom,

für einen Acylrest, der im Falle, daß $R_1$ die Bedeutung von Benzyl hat, mehr als zwei C-Atome enthalten soll oder für einen Alkylsulfonyl- oder Arylsulfonylrest steht oder

$R_1$ und $R_2$ gemeinsam mit dem Stickstoffatom Bestandteil eines gegebenenfalls substituierten heterocyclischen Ringes sind und

$R_3$ für Wasserstoff sowie einen Acyl- oder Alkylsulfonyl- oder Arylsulfonylrest stehen kann,

bei der Bekämpfung von Krankheiten.

9. Verwendung von Verbindungen der allgemeinen Formel I

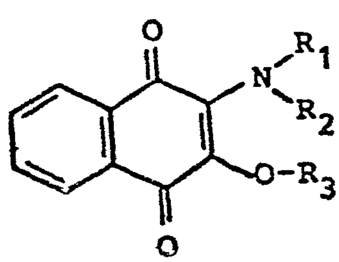

worin

$R_1$ für einen substituierten linearen oder verzweigten, gesättigten oder ungesättigten Alkylrest,

für einen gegebenenfalls substituierten gesättigten oder ungesättigten Cycloalkylrest,

für einen gegebenenfalls substituierten gesättigten oder ungesättigten Aralkylrest,

für einen substituierten Arylrest oder

für einen gegebenenfalls substituierten gesättigten oder ungesättigten heterocyclischen Rest steht,

$R_2$ dieselbe Bedeutung wie $R_1$ haben und gleich oder von diesem verschieden sein kann oder

für ein Wasserstoffatom,

für einen Acylrest, der im Falle, daß $R_1$ die Bedeutung von Benzyl hat, mehr als zwei C-Atome enthalten soll oder für einen Alkylsulfonyl- oder Arylsulfonylrest steht oder

$R_1$ und $R_2$ gemeinsam mit dem Stickstoffatom Bestandteil eines gegebenenfalls substituierten heterocyclischen Ringes sind und

$R_3$ für Wasserstoff sowie einen Acyl- oder Alkylsulfonyl- oder Arylsulfonylrest stehen kann, als Entzündungshemmer und/oder Antiarthritika.

TP 72

Patentansprüche für AT

1. **Verfahren zur Herstellung von Verbindungen der allgemeinen Formel I**

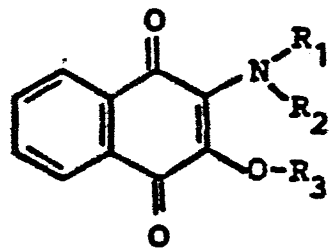

(I)

worin

$R_1$ für einen substituierten linearen oder verzweigten, gesättigten oder ungesättigten Alkylrest,

für einen gegebenenfalls substituierten gesättigten oder ungesättigten Cycloalkylrest,

für einen gegebenenfalls substituierten gesättigten oder ungesättigten Aralkylrest,

für einen substituierten Arylrest oder

für einen gegebenenfalls substituierten gesättigten oder ungesättigten heterocyclischen Rest steht,

$R_2$ dieselbe Bedeutung wie $R_1$ haben und gleich oder von diesem verschieden sein kann oder

für ein Wasserstoffatom,

für einen Acylrest, der im Falle, daß $R_1$ die Bedeutung von Benzyl hat, mehr als zwei C-Atome

enthalten soll oder für einen Alkylsulfonyl- oder Arylsulfonylrest steht oder

$R_1$ und $R_2$ gemeinsam mit dem Stickstoffatom Bestandteil eines gegebenenfalls substituierten heterocyclischen Ringes sind und

$R_3$ für Wasserstoff

dadurch gekennzeichnet, daß man
2-Epoxi-1,4-dioxotetralin der Formel II

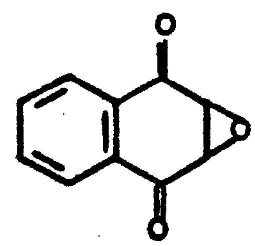

(II)

mit Aminen der allgemeinen Formel III

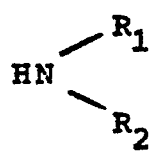

(III),

in der

$R_1$ für einen substituierten linearen oder verzweigten, gesättigten oder ungesättigten Alkylrest,

für einen gegebenenfalls substituierten gesättigten oder ungesättigten Cycloalkylrest,

für einen gegebenenfalls substituierten gesättigten oder ungesättigten Aralkylrest,

für einen substituierten Arylrest oder

für einen gegebenenfalls substituierten gesättigten oder ungesättigten heterocyclischen Rest steht,

und

$R_2$ dieselbe Bedeutung wie $R_1$ hat und gleich oder verschieden von diesem ist oder für ein Wasserstoffatom steht oder

$R_1$ und $R_2$ gemeinsam mit dem Stickstoffatom Bestandteil eines gegebenenfalls substituierten heterocyclischen Ringes sind

in einem geeigneten Lösungsmittel unter Bildung von Verbindungen der allgemeinen Formel IV umsetzt

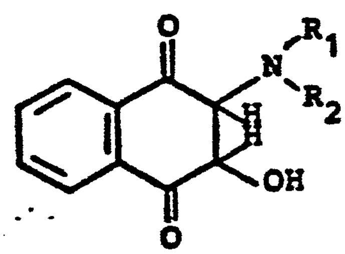

( IV )

und diese zu den Verbindungen der allgemeinen Formel I oxydiert.

2. Verfahren zur Herstellung von Verbindungen der allgemeinen Formel I

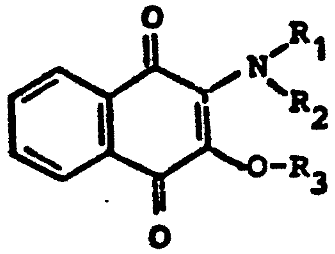

(I)

worin

$R_1$ für einen Alkylrest mit bis zu 6 C-Atomen, der durch Amino-, Mono-, Dialkyl-, Acylamino-, Alkoxy-, Acyloxy-, Alkylmercapto-, Carboxyl-, Carbamoyl-, Alkoxycarbonyl-, Hydroxyl-, Cyano-, Cycloalkylgruppen, aromatische oder teilweise oder ganz gesättigte Heterocyclen aus der Reihe Pyridyl, Furyl, Thienyl, Imdiazolyl, Pyrrolyl, Morpholinyl oder Indolyl substituiert sein kann,

für einen Cycloalkylrest mit bis zu 6 C-Atomen, der durch einen Alkylrest mit 1 bis 3 C-Atomen oder Phenylresten substituiert sein kann oder ein oder zwei Doppelbindungen enthalten kann,

für einen Aralkylrest, bei dem der Arylrest, vorzugsweise ein Phenylrest, der durch Hydroxyl-, Alkyl- oder Alkoxygruppen mit bis zu 3 C-Atomen, Halogenatome, Nitro-, Cyano-, Carboxyl-, Carbamoyl-, Alkoxycarbonyl-, Alkylmercapto-, Amino-, Mono- oder Dialkylamino, Acylamino oder Sulfamoylgruppen substituiert sein kann oder über eine Kohlenstoffkette mit bis zu 3 C-Atomen, die gesättigt sein oder eine Doppelbindung enthalten und gegebenenfalls durch niedrige Alkyl-, Alkoxy-, Acyloxy- oder Hydroxylgruppen oder einen Phenylrest substituiert sein kann, an das Stickstoffatom gebunden ist,

für einen Arylrest, bevorzugt einen Phenylrest, der durch niedrige Alkyl-, Alkylmercapto-, Acyl-, Acyloxy-, Acylamino-, Mono- oder Dialkylaminogruppen mit jeweils bis zu 3 C-Atomen, Fluoratome, Hydroxyl-, Amino-, Cyano-, Carbamoyl- oder Alkoxycarbonylgruppen substituiert sein kann,

für einen aromatischen oder teilweise oder ganz ungesättigten und gegebenenfalls einfach substituierten heterocyclischen fünf- oder sechsgliedrigen Ring, der als Heteroatome bis zu 4 Stickstoffatome und maximal je ein Sauerstoff- oder Schwefelatom enthalten kann, steht, und

$R_2$ dieselbe Bedeutung wie $R_1$ besitzt und gleich oder verschieden von diesem sein kann und für Wasserstoff oder einen geraden oder verzweigten, auch ungesättigten Acylrest mit bis zu 6 C-Atomen oder für einen Methan-, Ethan-, Benzol- oder Toluolsulfonylrest steht und

$R_1$ und $R_2$ gemeinsam mit dem Stickstoffatom Bestandteil eines gegebenenfalls substituierten heterocyclischen Ringes sind und

$R_3$ für Wasserstoff, einen geraden oder verzweigten, auch ungesättigten Acylrest mit 1 bis 6 C-Atomen, einen Methan-, Ethan-, Benzol- oder Toluolsulfonylrest steht,

dadurch gekennzeichnet, daß man 2-Epoxi-1,4-dioxo-tetralin der Formel II

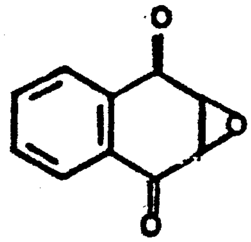

(II)

mit Aminen der allgemeinen Formel III

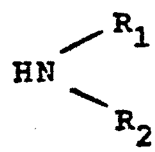

(III)

in der

$R_1$ und $R_2$ die oben angegebene Bedeutung haben, in einem geeigneten Lösungsmittel unter Bildung von Verbindungen der allgemeinen Formel IV

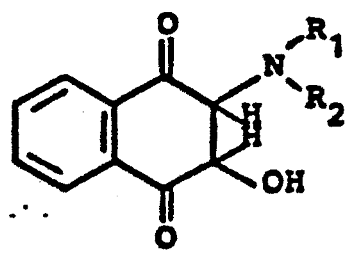

(IV)

in der

$R_1$ und $R_2$ die oben angegebene Bedeutung haben, umsetzt, und diese zu den Verbindungen der allgemeinen Formel I mit $R_3$ = H oxydiert und diese für den Fall, daß $R_3$ für Acyl oder einen Alkyl- oder Arylsulfonylrest steht, mit Säurederivaten, die diese Acyl- oder Aryl- oder Alkylsulfonylreste enthalten, umsetzt.

3. Verfahren zur Herstellung von 2-Benzyl-amino-3-hydroxy-naphthochinon-1,4, dadurch gekennzeichnet, daß man eine Suspension von 2-Epoxy-1,4-dioxotetralin in einem Lösungsmittel unter Rühren und Luftzutritt mit Benzylamin versetzt und nach beendeter Reaktion das Reaktionsgemisch mit einer Säure ansäuert, den ausgefallenen Niederschlag abfiltriert und aus einem geeigneten Lösungsmittel umkristallisiert.

4. Verfahren zur Herstellung von 2-(N-Acetyl-N-(2-furylmethyl)amino)-3-acetoxy-naphthochinon-1,4, dadurch gekennzeichnet, daß man eine Mischung aus 2-(2-furylmethyl)amino-3-hydroxy-naphthochinon-1,4,-Pyridin und Essigsäureanhydrid erwärmt, den nach Abdampfen des Lösungsmittels verbleibenden Rückstand chromatographiert, die Produkt-enthaltenden Fraktionen eindampft und den Rückstand mit Äther ausrührt.

5. Verfahren nach einem der Ansprüche 1 und 2, dadurch gekennzeichnet, daß man die Umsetzung des 2-Epoxy-1,4-dioxotetralins der Formel II mit Aminen der allgemeinen Formel III in einem polaren protischen Lösungsmittel durchführt.

6. Verfahren nach einem der Ansprüche 1, 2 und 5, dadurch gekennzeichnet, daß man die Reaktion bei Temperaturen zwischen 0 °C und der Rückflußtemperatur des jeweils verwendeten Lösungsmittels durchführt.

7. Verfahren nach Anspruch 1, dadurch gekennzeichnet, daß man durch gezielt unvollständige Umsetzung Verbindungen der Formel I erhält, in denen

$R_1$ für einen substituierten linearen oder verzweigten, gesättigten oder ungesättigten Alkylrest,
für einen gegebenenfalls substituierten, gesättigten oder ungesättigten Alkylrest,
für einen gegebenenfalls substituierten, gesättigten oder ungesättigten Cycloalkylrest,
für einen gegebenenfalls substituierten, gesättigten oder ungesättigten Aralkylrest,
für einen substituierten Arylrest oder
für einen gegebenenfalls substituierten, gesättigten oder ungesättigten heterocyclischen Rest steht,

$R_2$ für einen Acyl-, Alkylsulfonyl- oder Arylsulfonylrest, und

$R_3$ für Wasserstoff steht.

8. Verfahren zur Herstellung von Verbindungen der allgemeinen Formel I

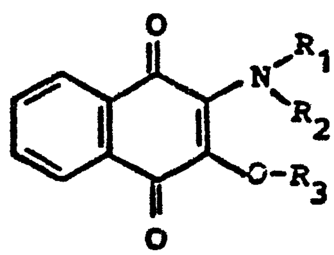 (I)

in denen

$R_1$ für einen substituierten linearen oder verzweigten, gesättigten oder ungesättigten Alkylrest,
für einen gegebenenfalls substituierten, gesättigten oder ungesättigten Alkylrest,
für einen gegebenenfalls substituierten, gesättigten oder ungesättigten Cycloalkylrest,
für einen gegebenenfalls substituierten, gesättigten oder ungesättigten Aralkylrest,
für einen substituierten Arylrest oder
für einen gegebenenfalls substituierten, gesättigten oder ungesättigten heterocyclischen Rest steht,

$R_2$ für einen Acyl-, Alkylsulfonyl- oder Arylsulfonylrest, und

$R_3$ für Wasserstoff steht.

dadurch gekennzeichnet, daß man Verbindungen der allgemeinen Formel I, in denen

$R_1$ für einen substituierten linearen oder verzweigten, gesättigten oder ungesättigten Alkylrest,
für einen gegebenenfalls substituierten, gesättigten oder ungesättigten Cycloalkylrest,
für einen gegebenenfalls substituierten, gesättigten oder ungesättigten Aralkylrest,
für einen substituierten Arylrest
oder für einen gegebenenfalls substituierten, gesättigten oder ungesättigten heterocyclischen Rest steht,
und

$R_2$ und $R_3$ für einen Acyl-, Alkylsulfonyl- oder Arylsulfonylrest stehen, nach an sich bekannten Methoden partiell verseift.

9. Verfahren nach Anspruch 1, dadurch gekennzeichnet, daß man die Oxidation der Verbindung der Formel IV zur Verbindung der Formel I mit Luftsauerstoff durchführt.

10. Verfahren nach Anspruch 1, dadurch gekennzeichnet, daß man die Verbindung IV vor der Oxidation isoliert.